# EUROPÄISCHE PATENTSCHRIFT

(11) **EP 1 045 827 B1**
(45) Veröffentlichungstag und Bekanntmachung des Hinweises auf die Patenterteilung: **10.09.2003**
(21) Anmeldenummer: 98966692.0
(22) Anmeldetag: 30.12.1998
(51) Int. Cl.: C07C 227/18, C07C 229/26, C07C 303/24, C07C 305/04

(54) **VERFAHREN ZUR HERSTELLUNG VON (S,S)-N,N'-ETHYLENDIAMINDIBERNSTEINSÄURE, ANALOGEN VERBINDUNGEN ODER SALZEN DAVON**
METHOD FOR PRODUCING (S,S)-N,N'-ETHYLENEDIAMINE DISUCCINIC ACID, ANALOGOUS COMPOUNDS OR SALTS THEREOF
PROCEDE DE PRODUCTION D'ACIDES (S,S)-N,N'-ETHYLENDIAMINODISUCCINIQUES, LEURS COMPOSES ANALOGUES OU LEURS SELS

(30) Priorität: 08.01.1998 DE 19800437
(43) Veröffentlichungstag der Anmeldung: 25.10.2000
(73) Patentinhaber: BASF AKTIENGESELLSCHAFT, 67056 Ludwigshafen (DE)
(72) Erfinder: OFTRING, Alfred, D-67098 Bad Dürkheim (DE); OTT, Christian, D-67346 Speyer (DE); POTTHOFF-KARL, Birgit, D-67061 Ludwigshafen (DE)
(74) Vertreter: Isenbruck, Günter
(86) Internationale Anmeldenummer: EP9808519
(87) Internationale Veröffentlichungsnummer: WO99035121

(56) Entgegenhaltungen:
- WO-A-96/01803
- WO-A-96/32371
- DE-A- 2 220 295

## Beschreibung

Die Erfindung betrifft ein Verfahren zur Herstellung von (S,S)-N,N'-Ethylendiamindibernsteinsäure ((S,S)-EDDS), analogen Verbindungen oder ihren Salzen. (S,S)-EDDS weist die folgende Formel auf: 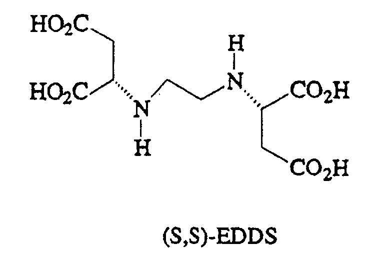 (S,S)-EDDS wird als biologisch abbaubarer Komplexbildner für Erdalkali- und Schwermetallionen verwendet. Verschiedene Verfahren zu ihrer Herstellung sind bekannt.

Beispielsweise kann die Herstellung von (S,S)-EDDS durch Mikroorganismen erfolgen. So können Alkylendiamine und Fumarsäure in Gegenwart von Burkholderia sp. umgesetzt werden, wie es in EP-A-0 731 171 beschrieben ist.

Die Herstellung kann auch durch Umsetzung von L-Asparaginsäure mit Glyoxal in Gegenwart von Basen und anschließender Reduktion mit Natriumborhydrid erfolgen. Ein derartiges Verfahren ist beispielsweise in WO 96/32371 beschrieben.

In der GB-A-2 299 809 ist die Umsetzung von L-Asparaginsäuremethylester mit 1,2-Dibromethan beschrieben.

Gemäß WO 96/01801 kann die Herstellung von (S,S)-EDDS durch Umsetzung von L-Asparaginsäure mit 1,2-Dibromethan in Wasser erfolgen.

Gemäß WO 94/28464 erfolgt die Herstellung von (rac)-EDDS durch Umsetzung von Ethylendiamin und Maleinsäure.

Die mikrobiellen Verfahren zur Herstellung von (S,S)-EDDS sind technisch aufwendig und liefern das gewünschte Produkt nur in geringer Ausbeute. Die anderen bekannten Verfahren liefern das gewünschte Produkt ebenfalls nur in geringer Ausbeute. Oft ist der Einsatz kostspieliger Chemikalien notwendig. Beim Einsatz von halogenhaltigen organischen Verbindungen entsteht immer ein gewisser Anteil an organisch gebundenem Halogen (AOX). Die Bildung von AOX sollte aus Gründen des Umweltschutzes möglichst vermieden werden.

Aufgabe der vorliegenden Erfindung ist die Bereitstellung eines Verfahrens zur Herstellung von (S,S)-EDDS und analogen Verbindungen, das aus kostengünstigen, leicht verfügbaren Ausgangsstoffen in einem unaufwendigen Verfahren (S,S)-EDDS in hoher Ausbeute liefert.

Die Aufgabe wird erfindungsgemäß gelöst durch ein Verfahren zur Herstellung von (S,S)-N,N'-Ethylendiamindibernsteinsäure, -diglutarsäure oder -disulfopropionsäure, (S,S)-N,N'-(2-Hydroxy)propylen-1,3-diamindibernsteinsäure, -diglutarsäure oder -disulfopropionsäure oder ihren Salzen durch Umsetzung von L-Asparaginsäure, L-Glutaminsäure oder L-Cysteinsäure mit 1,2-Bissulfooxyethan, 1,3,2-Dioxathiolan-2,2-dioxid oder deren Gemischen oder 1,3-Bisulfooxy-2-hydroxypropan in Gegenwart einer Base und gegebenenfalls anschließendes Ansäuern mit einer Säure.

Zudem betrifft die Erfindung ein Verfahren zur Herstellung von N-(2-Sulfooxyethyl)-Lasparaginsäure, -L-glutaminsäure oder -L-cysteinsäure, N-(3-Sulfooxy-2-hydroxypropyl)-L-asparaginsäure, -L-glutaminsäure oder -L-cysteinsäure oder ihren Salzen durch Umsetzung von L-Asparaginsäure, L-Glutaminsäure oder L-Cysteinsäure mit einer äquimolaren Menge an 1,2-Bissulfooxyethan, 1,3,2-Dioxythiolan-2,2-dioxid oder deren Gemischen oder 1,3-Bissulfooxy-2-hydroxypropan in Gegenwart einer Base und gegebenenfalls anschließendes Ansäuern mit einer Säure.

Es wurde erfindungsgemäß gefunden, dass die Herstellung von (S,S)-EDDS durch Umsetzung von L-Asparaginsäure mit Ethylenglykoldischwefelsäure (1,2-Bissulfooxyethan, CAS: 6913-91-6) nach folgendem Reaktionsschema erfolgen kann: 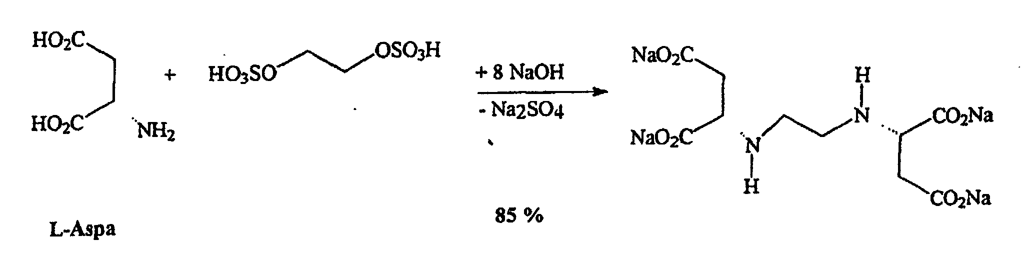 1,2-Bissulfooxyethan kann dabei durch Umsetzung von Ethylenglykol mit Schwefeltrioxid, Chlorsulfonsäure, Schwefelsäureanhydrid oder Schwefelsäure erhalten werden. Die Synthese ist beispielsweise in J. Prakt. Chem. 1879, 20, 2 beschrieben. Die Umsetzung von Schwefeltrioxid mit Ethylenglykol ist beispielsweise in J. Am. Chem. Soc. 1954, 76, 5361 beschrieben.

Anstelle von 1,2-Bissulfooxyethan kann auch das cyclische Glykolsulfat (1,3,2-Dioxathiolan-2,2-dioxid, CAS: 1072-53-3) eingesetzt werden. Glykolsulfat kann beispielsweise nach dem in DE-A-20 40 503 beschriebenen Verfahren hergestellt werden. Die Umsetzung erfolgt nach folgendem Schema:

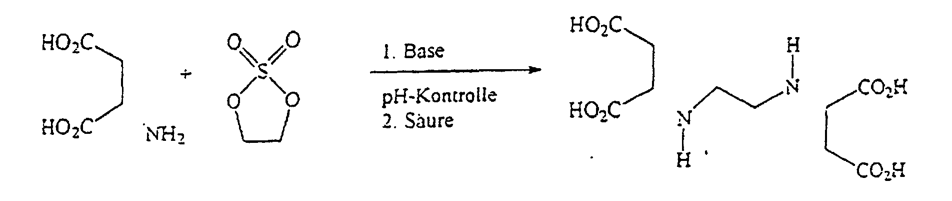

Anstelle von 1,2-Bissulfooxyethan oder 1,3,2-Dioxathiolan-2,2-dioxid kann auch ein Gemisch beider Verbindungen eingesetzt werden. Die Umsetzung erfolgt mit 2 Äquivalenten L-Asparaginsäure. Zudem kann die Umsetzung auch sequentiell durchgeführt werden. Dabei wird zunächst L-Asparaginsäure mit einem Äquivalent an 1,2-Bissulfooxyethan oder 1,3,2-Dioxathiolan-2,2-dioxyd oder Gemischen davon zu N-(2-Sulfooxyethyl)-L-asparaginsäure oder ihren Salzen umgesetzt. Sodann wird diese Verbindung mit einem Äquivalent L-Asparaginsäure in Gegenwart von Basen zu (S,S)-EDDS nach folgendem Schema umgesetzt:

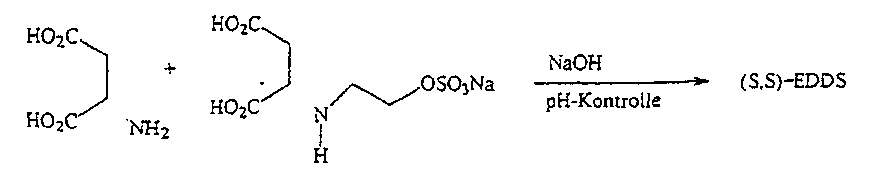

Als Base können in den Umsetzungen alle geeigneten Basen eingesetzt werden. Beispiele sind Alkali- und Erdalkalihydroxide, Alkali- und Erdalkalicarbonate, tertiäre Amine oder Ammoniak sowie Gemische davon. Vorzugsweise werden Natriumhydroxid, Natriumcarbonat, Kaliumhydroxid, Kaliumcarbonat oder Calciumhydroxid eingesetzt. Bei der Verwendung von Ammoniak kann entstehendes Ammoniumsulfat in einfacher Weise in Ammoniak und Schwefelsäure rücküberführt werden.

Bei der Umsetzung wird vorzugsweise der pH-Wert im Bereich von 7 bis 13, bevorzugt 7 bis 11, besonders bevorzugt von 8 bis 10, gehalten. Nach der Umsetzung kann das Produkt mit einer Säure angesäuert werden. Geeignete Säuren sind beispielsweise organische Carbonsäuren wie Essigsäure oder anorganische Säuren wie Schwefelsäure. Wird das Produkt vollständig angesäuert, so wird freies (S,S)-EDDS erhalten. Dieses kann durch mechanische Abtrennung (Filtration) isoliert werden, da es aus dem Reaktionsgemisch ausfällt. In diesem Fall liegen alle Carboxylgruppen im (S,S)-EDDS in protonierter Form vor. Durch Verzicht auf das Ansäuern mit einer Säure oder Ansäuern mit geringerer Menge an Säure können Salze von (S,S)-EDDS erhalten werden, in denen 1 bis 4 der Carboxylgruppen in Salzform vorliegen. So sind beispielsweise Mono-, Di-, Tri- oder Tetrasalze herstellbar. In den Salzen liegt als Gegenion das Ion der entsprechenden verwendeten Base vor. Bei Verwendung von Natriumcarbonat oder Natriumhydroxid können so Mono-, Di-, Tri- und Tetranatriumsalze von (S,S)-EDDS erhalten werden. Bei Verwendung der entsprechenden Kaliumverbindungen werden die entsprechenden Kaliumsalze erhalten. In Produktgemischen können auch im Mittel ungeradzahlige Werte für die Anzahl an Salzgruppen im Molekül erhalten werden.

Bei Verwendung von Calciumhydroxid als Base werden zunächst die entsprechenden Calciumsalze erhalten, die durch Versetzen mit Natriumcarbonat und Abtrennung des schwerlöslichen Calciumcarbonats in die Natriumsalze überführt werden können.

Die Umsetzung wird vorzugsweise in wäßriger Lösung durchgeführt. Dabei wird vorzugsweise L-Asparaginsäure in Wasser suspendiert und mit Base versetzt, bis sich der gewünschte pH-Wert einstellt. Sodann werden 1,2-Bissulfooxyethan, 1,3,2-Dioxathiolan-2,2-dioxid oder Gemische davon allmählich in das Reaktionsgemisch eingetragen. Bei der Umsetzung sollte die Reaktionstemperatur vorzugsweise 50°C, besonders bevorzugt 30°C, insbesondere 25°C nicht überschreiten. Während der Zugabe wird dabei der pH-Wert durch Zugabe von Base auf dem gewünschten Wert gehalten. Nach beendeter Zugabe wird das Reaktionsgemisch vorzugsweise auf eine Temperatur im Bereich von 30 bis 100°C, besonders bevorzugt 60 bis 100°C, insbesondere unter Rückfluß erhitzt. Das Fortschreiten der Umsetzung kann beispielsweise durch HPLC (High Performance Liquid Chromatography) verfolgt werden. Sobald keine weitere Abnahme der Menge an L-Asparaginsäure mehr nachweisbar ist, wird das Reaktionsgemisch abgekühlt und aufgearbeitet. Beispielswiese kann das Reaktionsgemisch mit Schwefelsäure sauer gestellt und das ausgefallene (S,S)-EDDS mechanisch abgetrennt werden. Das Abtrennen kann beispielsweise durch Filtrieren oder Zentrifugieren erfolgen. Das Reaktionsgemisch kann nach erfolgter Umsetzung auch ohne Ansäuern sprühgetrocknet werden. Es wird dabei ein Gemisch aus (S,S)-EDDS-tetranatriumsalz, L-Asparaginsäuredinatriumsalz, Ethylenglykol und Natriumsulfat erhalten, sofern als Base Natronlauge eingesetzt wurde. Bei Verwendung anderer Basen werden die entsprechenden Salze erhalten.

Entsprechende Umsetzungen sind mit L-Glutaminsäure, L-Cysteinsäure, 1,2-Bissulfooxyethan beziehungsweise 1,3-Bissulfooxy-2-hydroxypropan möglich. Es werden jeweils S,S-Konfigurationen erhalten, z.B. (S,S)-EDDG und (S,S)-HPDDS, d.h. (S,S)-Ethylendiamin-diglutaminsäure und 2-Hydroxypropylen-1,3-diamindibernsteinsäure.

Nachstehend wird die Erfindung anhand von Beispielen zusätzlich erläutert.

### Beispiele

### Beispiel 1

### (S,S)-N,N'-Ethylendiamindibernsteinsäure

In einer 1 l - Rührapparatur mit Rückflußkühler wurden 66,6 g (0,50 mol) L-Asparaginsäure in 200 ml Wasser bei Raumtemperatur suspendiert. Mit 50%iger Natronlauge wurde ein pH-Wert von 9 bis 10 eingestellt. 111 g (0,50 mol) 1,2-Bissulfooxyethan wurden langsam mit einem Feststofftrichter (Schneckentrichter) eingetragen, so daß die Innentemperatur 25°C nicht überschritt. Während der Zugabe wurde der pH-Wert mittels 50%iger Natronlauge bei 9 bis 10 gehalten.

Nach beendeter Zugabe wurde die Reaktionsmischung 4 h auf 100°C erwärmt. Die Reaktion wurde via HPLC verfolgt.

Sobald keine weitere Abnahme der L-Asparaginsäure mehr nachweisbar war, wurde mit 60%iger Schwefelsäure sauer gestellt und die ausgefallene (S,S)-N,N'-Ethylendiamindibernsteinsäure abgesaugt. Zur Reinigung wurde mit kaltem Wasser nachgewaschen. Ausbeute: 57,6 g (79%) (S,S)-N,N'-Ethylendiamindibernsteinsäure.

### Beispiel 2

### (S,S)-N,N' -Ethylendiamindibernsteinsäuretetranatriumsalz

In einer 1 l - Rührapparatur mit Rückflußkühler wurden 133 g (1 mol) L-Asparaginsäure in 400 ml Wasser bei Raumtemperatur suspendiert. Mit 50%iger Natronlauge wurde ein pH-Wert von 9 bis 10 eingestellt. 222 g (1 mol) 1,2-Bissulfooxyethan wurden langsam mit einem Feststofftrichter (Schneckentrichter) eingetragen, so daß die Innentemperatur 25°C nicht überschritt. Während der Zugabe wurde der pH-Wert mittels 50%iger Natronlauge bei 9 bis 10 gehalten.

Nach beendeter Zugabe wurde die Reaktionsmischung 4 h auf 100°C erwärmt. Die Reaktion wurde via HPLC verfolgt.

Sobald keine weitere Abnahme der Mengen an L-Asparaginsäure mehr nachweisbar war, wurde die Reaktionsmischung sprühgetrocknet. Man erhält eine Mischung aus 160 g (31 Gew.-% entspricht 84% chemische Ausbeute) (S,S)-N,N'-Ethylendiamindibernsteinsäuretetranatriumsalz, 28 g L-Asparaginsäuredinatriumsalz, 36 g Ethylenglykol und 284 g Natriumsulfat.

## Patentansprüche

1. Verfahren zur Herstellung von (S,S)-N,N'-Ethylendiamindibernsteinsäure, -diglutarsäure oder -disulfopropionsäure, (S,S)-N,N'-(2-Hydroxy)propylen-1,3-diamindibernsteinsäure, -diglutarsäure oder -disulfopropionsäure oder ihren Salzen durch Umsetzung von L-Asparaginsäure, L-Glutaminsäure oder L-Cysteinsäure mit 1,2-Bissulfooxyethan, 1,3,2-Dioxathiolan-2,2-dioxid oder deren Gemischen oder 1,3-Bisulfooxy-2-hydroxypropan in Gegenwart einer Base und gegebenenfalls anschließendes Ansäuern mit einer Säure.

2. Verfahren nach Anspruch 1, **dadurch gekennzeichnet, dass** ein bis vier der Carboxylgruppen in der (S,S)-N-N'-Ethylendiamindibernsteinsäure, -diglutarsäure oder -disulfopropionsäure, (S,S)-N,N'-(2-Hydroxy)propylen-1,3-diamindibernsteinsäure, -diglutarsäure oder -disulfopropionsäure in Salzform vorliegen.

3. Verfahren zur Herstellung von N-(2-Sulfooxyethyl)-L-asparaginsäure, -L-glutaminsäure oder -L-cysteinsäure, N-(3-Sulfooxy-2-hydroxypropyl)-Lasparaginsäure, -L-glutaminsäure oder -L-cysteinsäure oder ihren Salzen durch Umsetzung von L-Asparaginsäure, L-Glutaminsäure oder L-Cysteinsäure mit einer äquimolaren Menge an 1,2-Bissulfooxyethan, 1,3,2-Dioxythiolan-2,2-dioxid oder deren Gemischen oder 1,3-Bissulfooxy-2-hydroxypropan in Gegenwart einer Base und gegebenenfalls anschließendes Ansäuern mit einer Säure.

4. Verfahren nach einem der Ansprüche 1 bis 3, **dadurch gekennzeichnet, dass** als Base Alkalihydroxide, Alkalicarbonate, tertiäre Amine, Ammoniak oder Gemische davon eingesetzt werden.

5. Verfahren nach Ansprüche I bis 4, **dadurch gekennzeichnet, dass** während der Umsetzung der pH-Wert im Bereich von 8 bis 11 gehalten wird.

6. Verfahren nach einem der Ansprüche 1 bis 5, **dadurch gekennzeichnet, dass** das Produkt sprühgetrocknet oder nach Ansäuern durch mechanische Abtrennung isoliert wird.

## Claims

1. A process for the preparation of (S,S)-N,N'-ethylenediaminedisuccinic acid, (S,S)-N,N'-ethylenediaminediglutaric acid or (S,S)-N,N'-ethylenediaminedisulfopropionic acid, (S,S)-N,N'-(2-hydroxy)propylene-1,3-diaminedisuccinic acid, (S,S)-N,N'-(2-hydroxy)propylene-1,3-diaminediglutaric acid or (S,S)-N,N'-(2-hydroxy)propylene-1,3-diaminedisulfopropionic acid or their salts by reaction of L-aspartic acid, L-glutamic acid or L-cysteic acid with 1,2-bissulfooxyethane, 1,3,2-dioxathiolane 2,2-dioxide or mixtures thereof or 1,3-bissulfooxy-2-hydroxypropane in the presence of a base, and optional subsequent acidification with an acid.

2. A process as claimed in claim 1, wherein one to four of the carboxyl groups in the (S,S)-N,N'-ethylenediaminedisuccinic acid, (S,S)-N,N'-ethylenediaminediglutaric acid or (S,S)-N,N'-ethylenediaminedisulfopropionic acid, (S,S)-N,N'-(2-hydroxy)propylene-1,3-diaminedisuccinic acid, (S,S)-N,N'-(2-hydroxy)propylene-1,3-diaminediglutaric acid or (S,S)-N,N'-(2-hydroxy)propylene-1,3-diaminedisulfopropionic acid are present in salt form.

3. A process for the preparation of N-(2-sulfooxy-ethyl)-L-aspartic acid, N-(2-sulfooxyethyl)-L-glutamic acid or N-(2-sulfooxyethyl)-L-cysteic acid, N-(3-sulfooxy-2-hydroxypropyl)-L-aspartic acid, N-(3-sulfooxy-2-hydroxypropyl)-L-glutamic acid or N-(3-sulfooxy-2-hydroxypropyl)-L-cysteic acid or their salts by reaction of L-aspartic acid, L-glutamic acid or L-cysteic acid with an equimolar amount of 1,2-bissulfooxyethane, 1,3,2-dioxathiolane 2,2-dioxide or mixtures thereof or 1,3-bissulfooxy-2-hydroxypropane in the presence of a base, and optional subsequent acidification with an acid.

4. A process as claimed in any of claims 1 to 3, wherein the base used is an alkali metal hydroxide, alkali metal carbonate, tertiary amine, ammonia or a mixture thereof.

5. A process as claimed in claims 1 to 4, wherein the pH is maintained in the range from 8 to 11 during the reaction.

6. A process as claimed in any of claims 1 to 5, wherein the product is spray-dried or, after acidification, is isolated by mechanical separation.

## Revendications

1. Procédé de préparation d'acide (S,S)-N,N'-éthylènediamino-disuccinique, -diglutarique ou -disulfopropionique, d'acide (S,S)-N,N'-(2-hydroxy)propylène-1,3-diamino-disuccinique, -diglutarique ou -disulfopropionique ou de leurs sels au moyen d'une réaction de L-asparagine, d'acide L-glutamique ou de L-cystéine avec du 1,2-bissulfooxyéthane, du 2,2-dioxyde de 1,3,2-dioxathiolane ou des mélanges de ceux-ci ou du 1,3-bissulfooxy-2-hydroxy-propane en présence d'une base et, éventuellement, d'une acidification ultérieure avec un acide.

2. Procédé selon la revendication 1, **caractérisé en ce qu'**un à quatre des groupes carboxyle présents dans l'acide (S,S)-N,N'-éthylènediamino-disuccinique, -diglutarique ou -disulfopropionique, dans l'acide (S,S)-N,N'-(2-hydroxy)-propylène-1,3-diamino-disuccinique, -diglutarique ou -disulfopropionique sont présents sous forme de sel.

3. Procédé de préparation de N-(2-sulfooxyéthyl)-L-asparagine, d'acide L-glutamique ou de L-cystéine, de N-(3-sulfooxy-2-hydroxypropyl)-L-asparagine, d'acide L-glutamique ou de L-cystéine ou de leurs sels au moyen d'une réaction de L-asparagine, d'acide L-glutamique ou de L-cystéine avec une quantité équimolaire de 1,2-bissulfooxyéthane, de 2,2-dioxyde de 1,3,2-dioxathiolane ou de mélanges de ceux-ci ou de 1,3-bissulfooxy-2-hydroxypropane en présence d'une base et, éventuellement, d'une acidification ultérieure avec un acide.

4. Procédé selon l'une quelconque des revendications 1 à 3, **caractérisé en ce que** l'on utilise, en tant que base, des hydroxydes de métaux alcalins, des carbonates de métaux alcalins, des amines tertiaires, de l'ammoniaque ou des mélanges de ceux-ci.

5. Procédé selon les revendications 1 à 4, **caractérisé en ce que** l'on ajuste le pH pendant la réaction à des valeurs allant de 8 à 11.

6. Procédé selon l'une quelconque des revendications 1 à 5, **caractérisé en ce que** l'on sèche le produit par pulvérisation ou que l'on isole le produit par séparation mécanique après l'acidification.
